# EUROPEAN PATENT APPLICATION

(11) **EP 4 671 241 A1**
(43) Date of publication of application: **31.12.2025**
(21) Application number: 23924274.6
(22) Date of filing: 20.02.2023
(51) Int. Cl.: C07D 471/04, A61K 31/437, A61P 31/18

(54) **CRYSTALLINE FORM OF PYRROLOPYRIDINE DERIVATIVE**

(71) Applicant: ST Pharm Co., Ltd., Siheung-si, Gyeonggi-do 15086 (KR)
(72) Inventor: KIM, Uk-Il, Ansan-si, Gyeonggi-do 15610 (KR); BANG, Hyung Tae, Ansan-si, Gyeonggi-do 15610 (KR)
(74) Representative: D Young & Co LLP
(86) International application number: PCT/KR2023/002409
(87) International publication number: WO 2024/177165

(57) **Abstract**

The present invention pertains to: a polymorph of a pyrrolopyridine derivative which is a non-catalytic site integrase inhibitor; and a method for producing same. The novel polymorph of the present invention exhibits excellent non-hygroscopicity and stability and is thus suitable for pharmaceutical preparations.

## Description

### [Technical Field]

The present invention relates to a crystalline form of a pyrrolopyridine derivative, which is a non-catalytic site integrase inhibitor. Further, the present invention relates to a method for producing the crystalline form of the pyrrolopyridine derivative.

### [Background Art]

Acquired Immunodeficiency Syndrome (AIDS) is caused by infection with human immunodeficiency virus (HIV). For the treatment of AIDS, enzyme inhibitors have been developed according to HIV's mechanism of action. Depending on a point of action, the enzyme inhibitors are classified into a nucleoside reverse transcriptase inhibitor (NRTI), a protease inhibitor (PI), a fusion inhibitor, and an integrase inhibitor. Since the reverse transcriptase inhibitor, protease inhibitor, and fusion inhibitor have problems such as side effects, drug interactions, drug resistance and so on, thus the development of integrase inhibitors is actively progressing.

The integrase inhibitors are categorized into catalytic site inhibitors and non-catalytic site inhibitors based on their mechanism of action. A representative drug of catalytic site integrase inhibitors is Raltegravir. The mechanism of non-catalytic site integrase inhibition was introduced by Ziger Debyser et al. (Frauke Christ, Zeger Debyser at al., Nature Chemical Biology, 2010, Vol. 6, 442), but no drugs have been successfully developed yet.

However, Raltegravir, a catalytic site integrase inhibitor, was also found to exhibit drug resistance. In the case of HIV, if the medication discontinuation occurs, the medication taken is no longer effective since the latent HIV is reactivated and drug resistance develops, and thus the development of non-catalytic site integrase inhibitors is being attempted as a medication capable of solving the development of drug resistance. In particular, a pyrrolopyridine derivative represented by the following Chemical Formula I is known as the non-catalytic site integrase inhibitor:

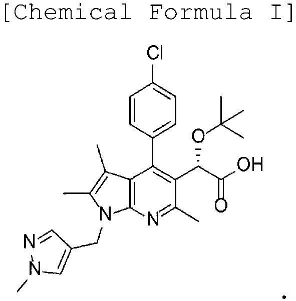

When designing a suitable dosage form in the pharmaceutical manufacturing process, it is important to select the physical state of the pharmaceutical raw material, i.e., crystalline form or amorphous form. However, crystalline raw materials have low solubility, which may result in low bioavailability per unit weight, and amorphous forms are unstable, resulting in difficulties in drug release and blood concentration control.

Therefore, it is necessary to develop a novel crystalline form of the pyrrolopyridine derivative represented by Chemical Formula I above that simultaneously solves these problems and exhibits excellent physicochemical properties.

### [Disclosure]

### [Technical Problem]

An object of the present invention is to provide a crystalline form of a pyrrolopyridine derivative having excellent non-hygroscopicity and stability and capable of improving solubility and bioavailability, and a method for producing the same.

### [Technical Solution]

In order to achieve the above purpose, the present inventors conducted research and made efforts to produce crystalline forms A to D described below, and confirmed that all of these crystalline forms exhibited excellent effects, thereby completing the present invention.

### Crystalline Form A and Production Method Thereof

The present invention provides a crystalline form A of a compound represented by the following Chemical Formula 1, which has a powder X-ray diffraction pattern comprising peaks at diffraction angles (2Θ±0.2°) of 9.26°, 10.47°, 12.76°, 14.70°, 15.83°, and 16.61°:

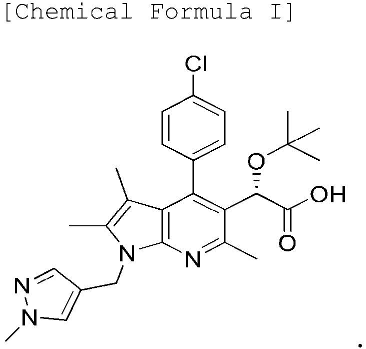

According to an embodiment of the present invention, the powder X-ray diffraction pattern of the crystalline form A may further comprise at least one peak at a diffraction angle (2θ±0.2') of 17.57°, 18.50°, 20.15°, 21.06°, 22.17°, 23.98°, or 27.05°.

According to an embodiment of the present invention, the powder X-ray diffraction pattern of the crystalline form A may further comprise at least one peak at a diffraction angle (2Θ±0.2°) of 8.02°, 8.39°, 8.72°, 13.92°, 16.40°, 17.79°, 19.33°, 23.38°, 24.36°, 24.90°, 25.59°, 25.99°, 26.70°, 27.85°, 29.37°, 29.98°, 30.77°, or 31.59°.

According to an embodiment of the present invention, the crystalline form A may have a differential scanning calorimetry (DSC) endothermic transition peak at 208 to 215 °C when the heating rate is 10 °C/min.

In addition, the present invention provides a method for producing the crystalline form A. Specifically, the above production method may comprise the following steps:
(A-1) adding the compound represented by Chemical Formula 1 above to toluene, ethyl acetate or isopropanol, followed by stirring; and
(A-2) filtering and drying the resulting solid.

### Crystalline Form B and Production Method Thereof

The present invention provides a crystalline form B of the compound represented by Chemical Formula 1 above, which has a powder X-ray diffraction pattern comprising peaks at diffraction angles (2Θ±0.2°) of 9.24°, 12.79°, 15.43°, 16.32°, 17.83°, and 19.35°.

According to an embodiment of the present invention, the powder X-ray diffraction pattern of the crystalline form B may further comprise at least one peak at a diffraction angle (2Θ±0.2°) of 20.18°, 23.35°, 23.84°, 24.46°, 26.03°, 29.66°, or 30.53°.

According to an embodiment of the present invention, the powder X-ray diffraction pattern of the crystalline form B may further comprise at least one peak at a diffraction angle (2θ±0.2°) of 7.94°, 10.03°, 11.65°, 14.63°, 16.80°, 17.55°, 18.63°, 21.58°, 22.67°, 23.62°, 25.29°, 27.15°, 28.13°, 29.38°, 30.68°, 31.61°, 32.05°, 33.04°, 34.41°, 35.50°, or 36.26°.

According to an embodiment of the present invention, the crystalline form B may have a differential scanning calorimetry (DSC) endothermic transition peak at 213 to 218 °C when the heating rate is 10 °C/min.

In addition, the present invention provides a method for producing the crystalline form B. Specifically, the above production method may comprise the following steps:
(B-1) adding the compound represented by Chemical Formula 1 above to ethanol, followed by stirring; and
(B-2) filtering and drying the resulting solid.

### Crystalline Form C and Production Method Thereof

The present invention provides a crystalline form C of the compound represented by Chemical Formula 1 above, which has a powder X-ray diffraction pattern comprising peaks at diffraction angles (2Θ±0.2°) of 7.62°, 8.61°, 10.10°, 12.06°, 13.52°, and 15.72°.

According to an embodiment of the present invention, the powder X-ray diffraction pattern of the crystalline form C may further comprise at least one peak at a diffraction angle (2Θ±0.2°) of 17.35°, 18.73°, 19.62°, 22.60°, 23.50°, or 25.57°.

According to an embodiment of the present invention, the powder X-ray diffraction pattern of the crystalline form C may further comprise at least one peak at a diffraction angle (2Θ±0.2°) of 9.23°, 9.73°, 11.36°, 12.85°, 15.37°, 16.16°, 17.85°, 20.34°, 21.00°, 23.96°, 24.37°, 25.21°, 26.24°, 27.33°, 27.84°, 29.70°, 30.32°, 31.08°, 33.94°, or 35.18°.

According to an embodiment of the present invention, the crystalline form C may have a differential scanning calorimetry (DSC) endothermic transition peak at 124 to 143 °C and 207 to 213 °C when the heating rate is 10 °C/min.

In addition, the present invention provides a method for producing the crystalline form C. Specifically, the above production method may comprise the following steps:
(C-1) adding the compound represented by Chemical Formula 1 above to methanol, followed by stirring; and
(C-2) filtering and drying the resulting solid.

### Crystalline Form D and Production Method Thereof

The present invention provides a crystalline form D of the compound represented by Chemical Formula 1 above, which has a powder X-ray diffraction pattern comprising peaks at diffraction angles (2Θ±0.2°) of 8.70°, 11.42°, 13.62°, 15.51°, 16.70°, and 21.58°.

According to an embodiment of the present invention, the powder X-ray diffraction pattern of the crystalline form D may further comprise at least one peak at a diffraction angle (2Θ±0.2°) of 7.52°, 9.37°, 9.89°, 18.59°, or 23.84°.

According to an embodiment of the present invention, the powder X-ray diffraction pattern of the crystalline form D may further comprise at least one peak at a diffraction angle (2θ±0.2') of 8.24°, 10.72°, 12.02°, 12.38°, 15.01°, 15.93°, 17.95°, 19.91°, 20.62°, 21.29°, 22.71°, or 27.10°.

According to an embodiment of the present invention, the crystalline form D may have a differential scanning calorimetry (DSC) endothermic transition peak at 204 to 209 °C when the heating rate is 10 °C/min.

In addition, the present invention provides a method for producing the crystalline form D. Specifically, the above production method may comprise the following steps:
(D-1) adding the compound represented by Chemical Formula 1 above to acetone and water, followed by stirring under reflux;
(D-2) filtering the insoluble matter, adding water, and stirring; and
(D-3) filtering and drying the resulting solid.

### [Advantageous Effects]

The crystalline form of the present invention may have low hygroscopicity and excellent stability under accelerated and long-term storage conditions, and may be stably maintained without changes in amount over a long period of time, which is advantageous for pharmaceutical preparations.

Further, the crystalline form of the present invention may have high solubility and bioavailability, and thus may be usefully employed as a pharmaceutical raw material.

### [Description of Drawings]

FIG. 1 shows the powder X-ray diffraction (PXRD) results of crystalline form A according to Example 1 of the present invention.
FIG. 2 shows the differential scanning calorimetry (DSC) thermogram results of crystalline form A according to Example 1 of the present invention.
FIG. 3 shows the powder X-ray diffraction (PXRD) results of crystalline form B according to Example 2 of the present invention.
FIG. 4 shows the differential scanning calorimetry (DSC) thermogram results of crystalline form B according to Example 2 of the present invention.
FIG. 5 shows the powder X-ray diffraction (PXRD) results of crystalline form C according to Example 3 of the present invention.
FIG. 6 shows the differential scanning calorimetry (DSC) thermogram results of crystalline form C according to Example 3 of the present invention.
FIG. 7 shows the powder X-ray diffraction (PXRD) results of crystalline form D according to Example 4 of the present invention.
FIG. 8 shows the differential scanning calorimetry (DSC) thermogram results of crystalline form D according to Example 4 of the present invention.

### [Best Mode]

Hereinafter, the present invention will be described in more detail through Examples and Experimental Examples to facilitate understanding. However, the following Examples and Experimental Examples are only provided to illustrate the present invention, and the content of the present invention is not limited to these Examples. These Examples and Experimental Examples of the present invention are provided to more comprehensively illustrate the invention to a person of ordinary skill in the art.

The reagents and solvents described below were purchased from Sigma-Aldrich Korea, TCI, and Daejung Chemicals & Metals unless otherwise specified, and HLPC was measured using the 1000 series of Agilent Technologies, Inc.

In addition, the various solid forms of (S)-2-(tert-butoxy)-2-(4-(4-chlorophenyl)-2,3,6-trimethyl-1-((1-methyl-1H-pyrazol-4-yl)methyl)-1H-pyrrolo[2,3-b]pyridin-5-yl)acetic acid prepared by the following Example were identified by X-ray diffraction patterns using X-ray diffraction and differential scanning calorimetry (DSC) patterns using DSC.

Specifically, powder X-ray diffraction analysis was conducted using a BRUKER D8 Focus equipped with a rotational meter and a solid-state detector, with measurements performed under the conditions of an analysis range of 5° ~ 50°, a step of 0.020°, and a step time of 171.6 s, using Cu-Kα rays. In addition, the DSC measurement by differential scanning calorimetry (DSC) is performed at 50 ~ 300 °C at a temperature increasing rate of 10 °C/min using DSC3 from METTLER TOREDO.

### Preparation Example. Preparation of (S)-2-(tert-butoxy)-2-(4-(4-chlorophenyl)-2,3,6-trimethyl-1-((1-methyl-1H-pyrazol-4-yl)methyl)-1H-pyrrolo[2,3-b]pyridin-5-yl)acetic acid

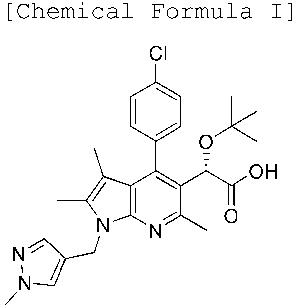

(S)-2-(tert-butoxy)-2-(4-(4-chlorophenyl)-2,3,6-trimethyl-1-((1-methyl-1H-pyrazol-4-yl)methyl)-1H-pyrrolo[2,3-b]pyridin-5-yl)acetate (13.5 g, 25.8 mmol) was diluted in 108 mL of tetrahydrofuran and 27 mL of methanol. Sodium hydroxide (3.1 g, 77.4 mmol) was added, and stirred at 40 ~ 45 °C for 4 hours. After the reaction was completed, the resulting mixture was concentrated under reduced pressure, and 120 mL of dichloromethane and 60 mL of purified water were added. The reaction mixture was cooled to 0 ~ 5 °C, and adjusted to pH 4.5 ~ 5.0 with 2N-hydrochloric acid solution. The organic layer was separated, and washed with 60 mL of purified water, then dried over anhydrous sodium sulfate, filtered, and concentrated under reduced pressure. To the obtained residue, 30 mL of acetonitrile was added, and the resulting mixture was cooled to 5 ~ 10 °C, and stirred for 3 hours. The precipitated crystals were filtered and dried under reduced pressure to obtain the title compound (9.25 g, 72%, ee: 99%) as an off-white solid.

### Example 1. Production of Crystalline Form A

The 1 g of (S)-2-(tert-butoxy)-2-(4-(4-chlorophenyl)-2,3,6-trimethyl-1-((1-methyl-1H-pyrazol-4-yl)methyl)-1H-pyrrolo[2,3-b]pyridin-5-yl)acetic acid prepared in Preparation Example above of the present invention was added to various solvents according to Table 1 below, followed by stirring at room temperature overnight. The resulting solid was filtered and dried in a hot air dryer at 50 °C to obtain crystalline form A.

**[Table 1]**

| Entry | Solvent | Yield | Crystalline Form |
|---|---|---|---|
| 1-1 | Toluene (10V) | 70% | Crystalline Form A |
| 1-2 | Ethyl Acetate (10V) | 50% | |
| 1-3 | Isopropanol (5V) | 76% | |

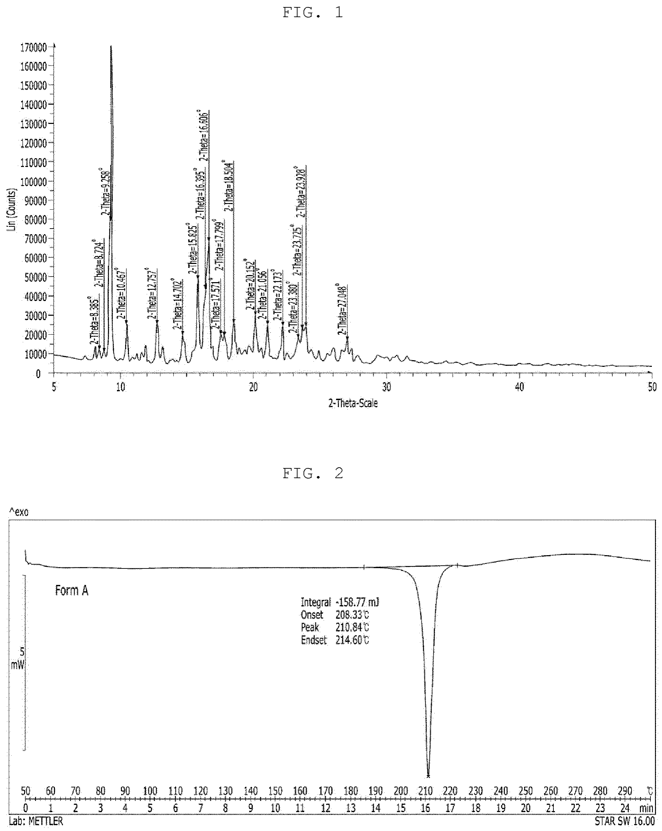

The powder X-ray diffraction (PXRD) peak and differential scanning calorimetry (DSC) endothermic peak for the obtained solid were analyzed, and the results are shown in FIGS. 1 and 2, respectively.

As confirmed in FIG. 1, the solid compound exhibited main peaks at 2θ (±0.2°) values of 9.26°, 10.47°, 12.76°, 14.70°, 15.83°, and 16.61°.

In addition, it was confirmed from FIG. 2 that the solid compound exhibited a differential scanning calorimetry (DSC) endothermic transition peak at 208 to 215 °C when the heating rate was 10 °C/min.

### Example 2. Production of Crystalline Form B

The 1 g of (S)-2-(tert-butoxy)-2-(4-(4-chlorophenyl)-2,3,6-trimethyl-1-((1-methyl-1H-pyrazol-4-yl)methyl)-1H-pyrrolo[2,3-b]pyridin-5-yl)acetic acid prepared in Preparation Example above of the present invention was added to ethanol (5 mL, 5 V), followed by stirring at room temperature overnight. The resulting solid was filtered and dried in a hot air dryer at 50 °C to obtain crystalline form B (0.71 g, 71%).

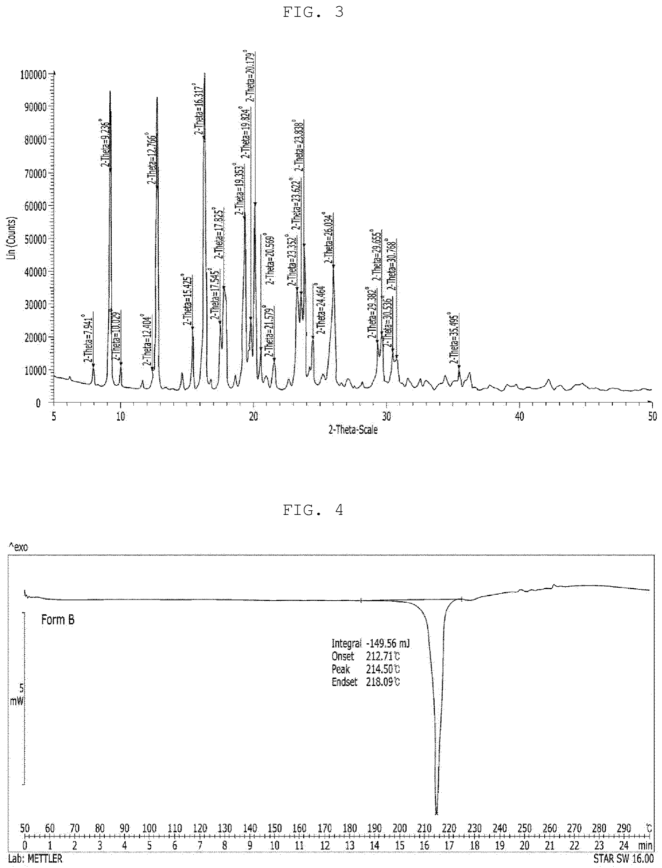

The powder X-ray diffraction (PXRD) peak and differential scanning calorimetry (DSC) endothermic peak for the obtained solid were analyzed, and the results are shown in FIGS. 3 and 4, respectively.

As confirmed in FIG. 3, the solid compound exhibited main peaks at 2θ (±0.2°) values of 9.24°, 12.79°, 15.43°, 16.32°, 17.83°, and 19.35°.

Further, it was confirmed from FIG. 4 that the solid compound exhibited a differential scanning calorimetry (DSC) endothermic transition peak at 213 to 218 °C when the heating rate was 10 °C/min.

### Example 3. Production of Crystalline Form C

The 1 g of (S)-2-(tert-butoxy)-2-(4-(4-chlorophenyl)-2,3,6-trimethyl-1-((1-methyl-1H-pyrazol-4-yl)methyl)-1H-pyrrolo[2,3-b]pyridin-5-yl)acetic acid prepared in Preparation Example above of the present invention was added to methanol (5 mL, 5 V), followed by stirring at room temperature overnight. The resulting solid was filtered and dried in a hot air dryer at 50 °C to obtain crystalline form C (0.71 g, 76%).

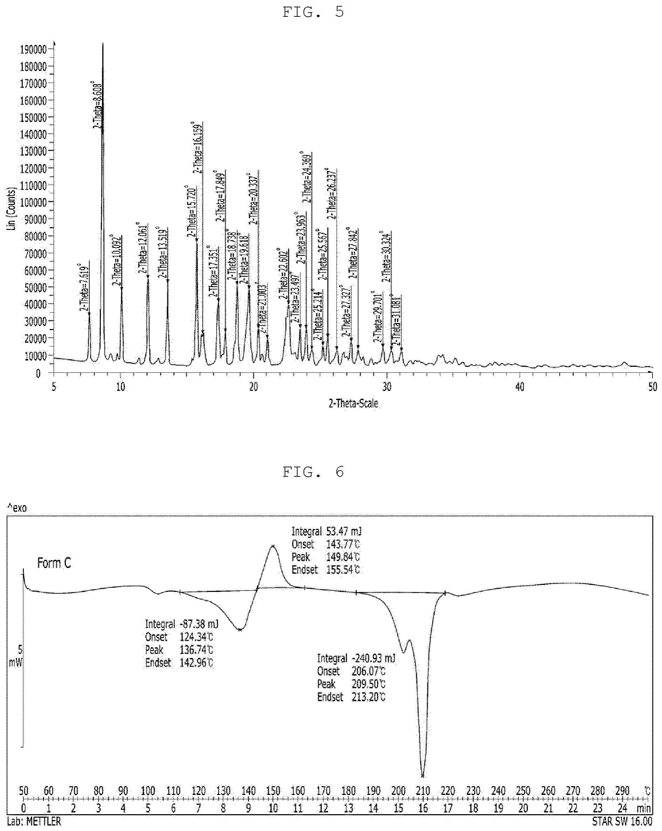

The powder X-ray diffraction (PXRD) peak and differential scanning calorimetry (DSC) endothermic peak for the obtained solid were analyzed, and the results are shown in FIGS. 5 and 6, respectively.

As confirmed in FIG. 5, the solid compound exhibited main peaks at 2θ (±0.2°) values of 7.62°, 8.61°, 10.10°, 12.06°, 13.52°, and 15.72°.

Further, it was confirmed from FIG. 6 that the solid compound exhibited a differential scanning calorimetry (DSC) endothermic transition peak at 124 to 143 °C when the heating rate was 10 °C/min. The exothermic peak observed after the endothermic (melting) peak indicates the precipitation of crystals. After the crystal precipitation (exothermic peak), the endothermic peak was observed again at 206 to 213 °C.

### Example 4. Production of Crystalline Form D

The 1 g of (S)-2-(tert-butoxy)-2-(4-(4-chlorophenyl)-2,3,6-trimethyl-1-((1-methyl-1H-pyrazol-4-yl)methyl)-1H-pyrrolo[2,3-b]pyridin-5-yl)acetic acid prepared in Preparation Example above of the present invention was diluted in a mixed solvent of 10 mL of acetone and 0.4 mL of purified water, followed by stirring under reflux for 1 hour. The insoluble matter that did not dissolve was filtered off, and 10 mL of purified water was added dropwise to the filtrate for 10 minutes with stirring. The resulting solid was stirred at room temperature for 30 minutes, filtered, and dried in a hot air dryer at 50 °C to obtain crystalline D (0.91 g, 91%).

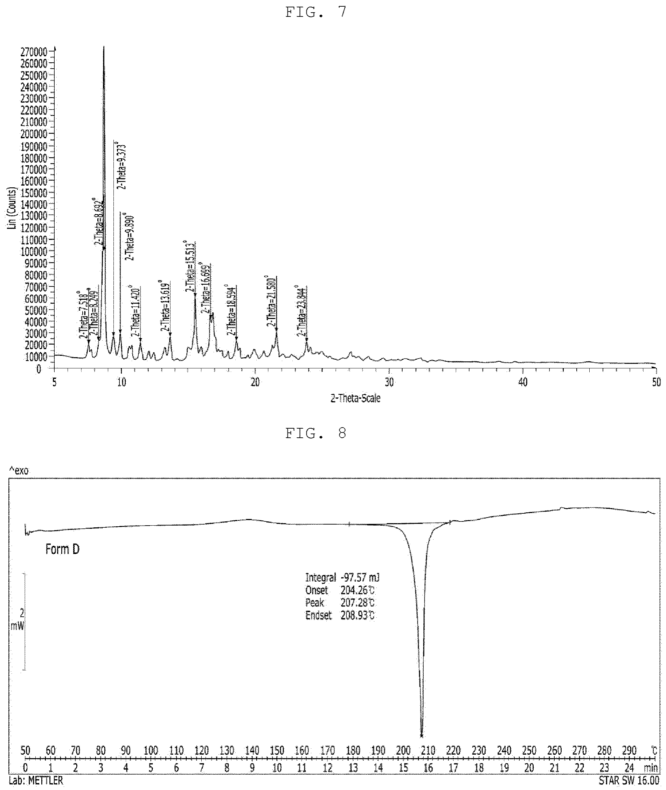

The powder X-ray diffraction (PXRD) peak and differential scanning calorimetry (DSC) endothermic peak for the solid were analyzed, and the results are shown in FIGS. 7 and 8, respectively.

As confirmed in FIG. 7, the solid compound exhibited main peaks at 2θ (±0.2°) values of 8.70°, 11.42°, 13.62°, 15.51°, 16.70°, and 21.58°.

In addition, it was confirmed from FIG. 8 that the solid compound exhibited a differential scanning calorimetry (DSC) endothermic transition peak at 204 to 209 °C when the heating rate was 10 °C/min.

### Experimental Example 1. Hygroscopicity (DVS) Measurement Experiment

The crystalline forms produced in Examples of the present invention were tested for hygroscopicity.
**a. Equipment Used in the Experiment**
   - Model Name: DVS Intrinsic (Surface Measurement Systems)
   - Specifications
      Temperature Range: 20 ~ 40 °C
      Temperature stability: ± 0.2 °C
      Humidity range: 0 ~ 98% RH
      RH Accuracy: ±1% RH
      Typical gas (Nitrogen + water vapor) flow rate: 200 sccm
      Sample chamber: 40mm wide x 50mm deep x 50mm high
      Heating system: Peltier + Cartridge
**b. Sample Information**
   Experiments were performed on the crystalline forms of Examples above.
**c. Experimental Conditions**
   - Temperature: 25 °C
   - Humidity range: Starting from 0%, reaching up to 95%
   - Solvent: Water
**d. Experimental Method**

A predetermined amount (10~20 mg) of the crystalline form prepared in each Example was placed on the sample pan in the sample chamber, and nitrogen gas was purged through the tube connected to the sample chamber for one hour to remove moisture from both the chamber and the sample. The humidity in the chamber was controlled by adjusting the water vapor, and the target relative humidity was increased from 0% to about 10% step by step to measure the moisture content of the crystalline forms. The mass of each sample was measured (from 0% RH to 95% RH) and the changes in sample weight resulting from an increase or decrease in humidity were plotted as a graph.

The change in moisture content of the crystalline forms according to Examples is as shown in Table 2 below.

**[Table 2]**

| Relative Humidity (%) | Crystalline Form A (% Change in Weight) | Crystalline Form B (% Change in Weight) |
|---|---|---|
| 0 | 0 | 0 |
| 10 | 0.02 | 0.01 |
| 20 | 0.05 | 0.02 |
| 30 | 0.07 | 0.03 |
| 40 | 0.10 | 0.04 |
| 50 | 0.15 | 0.05 |
| 60 | 0.21 | 0.07 |
| 70 | 0.27 | 0.09 |
| 80 | 0.34 | 0.10 |
| 90 | 0.45 | 0.13 |
| 95 | 0.51 | 0.15 |

As a result of the analysis, as shown in Table 2 above, the crystalline forms of the present invention were observed to have excellent non-hygroscopicity with almost no change in moisture content. This indicates that the crystalline form of the present invention is superior in formulation during the entire process of manufacturing, storage, and formulation of the active ingredient.

### Experimental Example 2. Accelerated Stability Test

The powder of crystalline form according to each Example of the present invention was double-packaged in a PE bag and triple-packaged in an Aluminum bag, and left at 40 ± 2°C, RH 75 ± 5% for 0 and 6 months, and then the impurities were measured using HPLC. The results are shown in Table 3 below.

**[Table 3]**

| Test Items | Example 1 | | Example 2 | |
|---|---|---|---|---|
| | 0 month (initial) | 6 month | 0 month (initial) | 6 month |
| Related Substances (%) | 0.17 | 0.23 | 0.03 | 0.06 |
| Assay (%) | 98.3 | 98.7 | 99.8 | 99.4 |
| Crystalline Form | Type A | Type A | Type B | Type B |

It was confirmed from the results in Table 3 above that the crystalline forms of the present invention remained crystalline, with no significant changes in related substances and amount observed. Therefore, it was appreciated that the crystalline forms of the present invention were able to stably maintain high purity for 6 months under accelerated conditions, showing excellent stability, little change in moisture content, and significantly lower hygroscopicity.

### Experimental Example 3. Long-Term Stability Test

The powder of crystalline form according to each Example of the present invention was double-packaged in a PE bag and triple-packaged in an Aluminum bag, and left at 25 ± 2°C, RH 60 ± 5% for 0 and 12 months, and then the impurities were measured using HPLC. The results are shown in Table 4 below.

**[Table 4]**

| Test Items | Example 1 | | Example 2 | |
|---|---|---|---|---|
| | 0 month (initial) | 12 month | 0 month (initial) | 12 month |
| Related Substances (%) | 0.17 | 0.22 | 0.03 | 0.03 |
| Assay (%) | 98.3 | 98.5 | 99.8 | 99.1 |
| Crystalline Form | Type A | Type A | Type B | Type B |

It was confirmed from the results in Table 4 above that the crystalline forms of the present invention remained crystalline, with no significant changes in related substances and amount observed. Therefore, it was appreciated that the crystalline forms of the present invention were able to stably maintain high purity for 12 months, showing excellent stability, little change in moisture content, and significantly lower hygroscopicity.

## Claims

1. A crystalline form A of a compound represented by the following Chemical Formula 1, which has a powder X-ray diffraction pattern comprising peaks at diffraction angles (2Θ±0.2°) of 9.26°, 10.47°, 12.76°, 14.70°, 15.83°, and 16.61°:[Chemical Formula I]

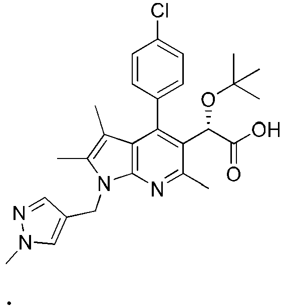

2. The crystalline form A of claim 1, wherein the powder X-ray diffraction pattern further comprises at least one peak at a diffraction angle (2θ±0.2°) of 17.57°, 18.50°, 20.15°, 21.06°, 22.17°, 23.98°, or 27.05°.

3. The crystalline form A of claim 2, wherein the powder X-ray diffraction pattern further comprises at least one peak at a diffraction angle (2Θ±0.2°) of 8.02°, 8.39°, 8.72°, 13.92°, 16.40°, 17.79°, 19.33°, 23.38°, 24.36°, 24.90°, 25.59°, 25.99°, 26.70°, 27.85°, 29.37°, 29.98°, 30.77°, or 31.59°.

4. The crystalline form A of claim 1, wherein the crystalline form A has a differential scanning calorimetry (DSC) endothermic transition peak at 208 to 215 °C when the heating rate is 10 °C/min.

5. A method for producing the crystalline form A of the compound represented by Chemical Formula 1 according to claims 1 to 4, comprising:
(A-1) adding the compound represented by the following Chemical Formula 1 to toluene, ethyl acetate or isopropanol, followed by stirring; and
(A-2) filtering and drying the resulting solid:

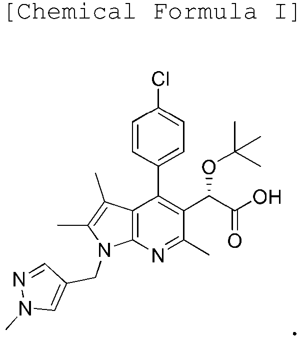

6. A crystalline form B of a compound represented by the following Chemical Formula 1, which has a powder X-ray diffraction pattern comprising peaks at diffraction angles (2θ±0.2') of 9.24°, 12.79°, 15.43°, 16.32°, 17.83°, and 19.35°.

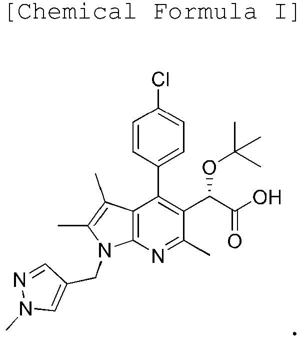

7. The crystalline form B of claim 6, wherein the powder X-ray diffraction pattern further comprises at least one peak at a diffraction angle (2θ±0.2') of 20.18°, 23.35°, 23.84°, 24.46°, 26.03°, 29.66°, or 30.53°.

8. The crystalline form B of claim 7, wherein the powder X-ray diffraction pattern further comprises at least one peak at a diffraction angle (2θ±0.2') of 7.94°, 10.03°, 11.65°, 14.63°, 16.80°, 17.55°, 18.63°, 21.58°, 22.67°, 23.62°, 25.29°, 27.15°, 28.13°, 29.38°, 30.68°, 31.61°, 32.05°, 33.04°, 34.41°, 35.50°, or 36.26°.

9. The crystalline form B of claim 6, wherein the crystalline form B has a differential scanning calorimetry (DSC) endothermic transition peak at 213 to 218 °C when the heating rate is 10 °C/min.

10. A method for producing the crystalline form B of the compound represented by Chemical Formula 1 according to claims 6 to 9, comprising:
(B-1) adding the compound represented by the following Chemical Formula 1 to ethanol, followed by stirring; and
(B-2) filtering and drying the resulting solid:

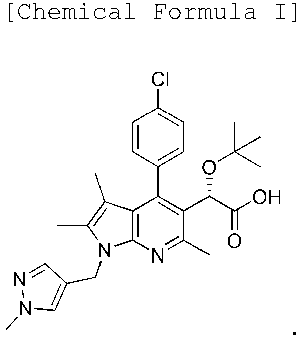

11. A crystalline form C of a compound represented by the following Chemical Formula 1, which has a powder X-ray diffraction pattern comprising peaks at diffraction angles (2θ±0.2') of 7.62°, 8.61°, 10.10°, 12.06°, 13.52°, and 15.72°:

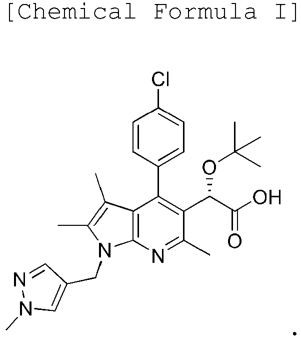

12. The crystalline form C of claim 11, wherein the powder X-ray diffraction pattern further comprises at least one peak at a diffraction angle (2Θ±0.2°) of 17.35°, 18.73°, 19.62°, 22.60°, 23.50°, or 25.57°.

13. The crystalline form C of claim 12, wherein the powder X-ray diffraction pattern further comprises at least one peak at a diffraction angle (2Θ±0.2°) of 9.23°, 9.73°, 11.36°, 12.85°, 15.37°, 16.16°, 17.85°, 20.34°, 21.00°, 23.96°, 24.37°, 25.21°, 26.24°, 27.33°, 27.84°, 29.70°, 30.32°, 31.08°, 33.94°, or 35.18°.

14. The crystalline form C of claim 11, wherein the crystalline form C has a differential scanning calorimetry (DSC) endothermic transition peak at 124 to 143 °C and 207 to 213 °C when the heating rate is 10 °C/min.

15. A method for producing the crystalline form C of the compound represented by Chemical Formula 1 according to claim 11to 14, comprising:
(C-1) adding the compound represented by the following Chemical Formula 1 to methanol, followed by stirring; and
(C-2) filtering and drying the resulting solid:

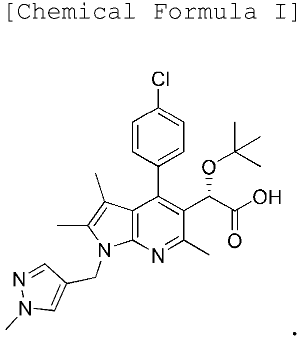

16. A crystalline form D of a compound represented by the following Chemical Formula 1, which has a powder X-ray diffraction pattern comprising peaks at diffraction angles (2θ±0.2') of 8.70°, 11.42°, 13.62°, 15.51°, 16.70°, and 21.58°:

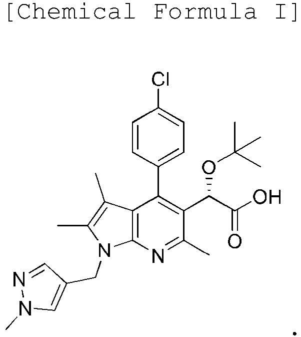

17. The crystalline form D of claim 16, wherein the powder X-ray diffraction pattern further comprises at least one peak at a diffraction angle (2Θ±0.2°) of 7.52°, 9.37°, 9.89°, 18.59°, or 23.84°.

18. The crystalline form D of claim 17, wherein the powder X-ray diffraction pattern further comprises at least one peak at a diffraction angle (2θ±0.2') of 8.24°, 10.72°, 12.02°, 12.38°, 15.01°, 15.93°, 17.95°, 19.91°, 20.62°, 21.29°, 22.71°, or 27.10°.

19. The crystalline form D of claim 16, wherein the crystalline form D has a differential scanning calorimetry (DSC) endothermic transition peak at 204 to 209 °C when the heating rate is 10 °C/min.

20. A method for producing the crystalline form D of the compound represented by Chemical Formula 1 according to claims 16 to 19, comprising:
(D-1) adding the compound represented by the following Chemical Formula 1 to acetone and water, followed by stirring under reflux;
(D-2) filtering the insoluble matter, adding water, and stirring; and
(D-3) filtering and drying the resulting solid:

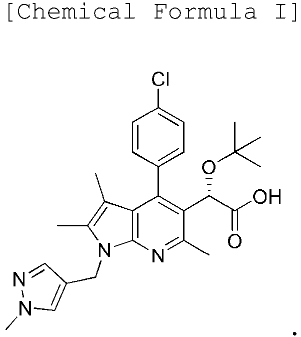

21. An antiviral pharmaceutical composition comprising: the crystalline form A according to claims 1 to 4, the crystalline form B according to claims 6 to 9, the crystalline form C according to claims 11 to 14, or the crystalline form D according to claims 16 to 19.

22. The antiviral pharmaceutical composition of claim 21, wherein the composition is for anti-human immunodeficiency virus (HIV).
